# EUROPEAN PATENT APPLICATION

(11) **EP 3 101 135 A1**
(43) Date of publication of application: **07.12.2016**
(21) Application number: 15170150.5
(22) Date of filing: 01.06.2015
(51) Int. Cl.: C12N 15/82, A01H 5/10

(54) **METHOD OF CONFERRING RESISTANCE AGAINST A FUSARIUM PLANT DISEASE**

(71) Applicant: Universität Hamburg, 20148 Hamburg (DE)
(72) Inventor: Jörg, Bormann, 22529 Hamburg (DE); Cornelia, Heinze, 22869 Schenefeld (DE); Wilhelm, Schäfer, 22589 Hamburg (DE)
(74) Representative: Stüven, Ralf

(57) **Abstract**

The invention relates to a method of conferring to a plant resistance against a plant disease caused by a *Fusarium* species, to a transgenic *Fusarium*-resistant plant, to a double-stranded RNA and a composition comprising same. Object of the invention is the provision of means for reducing susceptibility of food crops, in particular cereals, to fungal attack. For this purpose, the present invention provides in one aspect a method of conferring to a plant resistance against a plant disease caused by a *Fusarium* species, the method comprising genetically engineering the plant in a manner to enable host-induced gene silencing of a *Fusarium* gene encoding CSX1 having or comprising the amino acid sequence of SEQ ID NO: 2 or of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

## Description

The invention relates to a method of conferring to a plant resistance against a plant disease caused by a *Fusarium* species, to a transgenic *Fatsarium*-resistant plant, to a double-stranded RNA and a composition comprising same.

Infestation of cereals with the fungus *Fusarium,* in particular with *Fusarium graminearum*, causes high economic losses and is one of the greatest threats to the food supply for a growing world population (see e.g. Goswami RS, Kistler HC, 2004, Heading for disaster: Fusarium graminearum on cereal crops, Mol. Plant Pathol 5: 515-525). *Fusarium graminearum* causes Fusarium head blight (FHB), resulting in shrunken, light-weight white or orange coulored kernels called fusarium-damaged kernels (FDK).

Methods to improve resistance of plants to fungi and to *Fusarium graminearum* are generally known (see e.g. WO 2006/070227 A2; WO 2006/097465 A2; WO 2010/023491 A2; WO 2012/155109 A1; WO 2014/023285 A2; WO 2013/050410 A1; US 2011/0061128 A1; Chuntao Y, 2011, Development of a Host-Induced RNAi System in the Wheat Stripe Rust Fungus Puccinia striiformis f. sp. tritici, Molecular Plant-Microbe Interactions 24: 554-561; Cheng W, 2015 Host-induced gene silencing of an essential chitin synthase gene confers durable resistance to Fusarium head blight and seedling blight in wheat, Plant Biotechnol J., doi: 10.1111/pbi.12352). Presently, however, fully *Fusarium*-resistant cereals are not available, and in conventional agriculture *Fusarium* infections are therefore confined by large-scale spraying of fungicides, mainly azoles (Becher R et al. 2011, Development of a novel multiplex DNA microarray for Fusarium graminearum and analysis of azole fungicide responses, BMC Genomics 12: 52, doi:10.1186/1471-2164-12-52). This approach, however, is more and more limited due to the increasingly common development of resistances against fungicides (Becher R et al. (2010), Adaptation of Fusarium graminearum to Tebuconazole Yielded Descendants Diverging for Levels of Fitness, Fungicide Resistance, Virulence, and Mycotoxin Production, Phytopathology 100: 444-453).

There is still a need to reduce the susceptibility of food crops to fungal attack. It is therefore an object of the present invention to provide means for this purpose.

In a first aspect the present invention provides a method of conferring to a plant resistance against a plant disease caused by a *Fusarium* species, the method comprising genetically engineering the plant in a manner to enable host-induced gene silencing of a *Fusarium* gene encoding CSX1 having or comprising the amino acid sequence of SEQ ID NO: 2 or of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

The invention makes, for the first time, use of a previously unknown fungal defense mechanism against certain mycoviruses. Recently, it has been shown that infection of *Fusarium graminearum* with the dsRNA mycovirus FgV-Ch9 affects vegetative growth, sexual and asexual reproduction, and also the virulence of the fungus (Darissa et al., 2012, A dsRNA mycovirus causes hypovirulence of Fusarium graminearum to wheat and maize, European Journal of Plant Pathology 134: 181-189, DOI 10.1007/s10658-012-9977-5). High viral dsRNA levels were associated with reduced virulence for wheat and maize plants. It has now surprisingly been found that a fungal protein, which will be denoted CSX1 in the following and which amino acid sequence is given in SEQ ID NO: 2, plays a key role in a fungal defense mechanism against the virus. Astonishingly, *Fusarium* downregulates the expression of CSX1 in response to an infection with FgV-Ch9. Without wishing to be bound by any specific theory it is believed that fungal CSX1 expression determines the infection potential of the virus, and that the fungus downregulates CSX1 when detecting the presence of the virus as a countermeasure, although downregulating CSX1 is also associated with reduced fitness (hypovirulence) of the fungus itself.

The term "Fusarium" refers to a genus of filamentous fungi (see e.g. Watanabe M, 2011, Molecular phylogeny of the higher and lower taxonomy of the Fusarium genus and differences in the evolutionary histories of multiple genes, BMC Evolutionary Biology 11: 322, doi:10.1186/1471-2148-11-322). The term refers especially to plant pathogenic *Fusarium* species. Examples are *Fusarium graminearum* (*Gibberella zeae*), *Fusarium avenaceum*, *Fusarium culmorum*, *Fusarium poae*, and *Fusarium oxysporum*.

The term "resistance", as used herein in view of a plant trait, refers to the decreased susceptibility of a plant or plant population to a pest, e.g. fungal infestation. The term encompasses a decreased susceptibility in terms of the number of plants of a plant population affected by the pest, and/or the number, degree or duration of disease symptoms caused by the pest, compared to a susceptible wildtype plant or plant population. Thus, the term "resistance" is not restricted to meaning that an infection with a pest does not occur. Rather, it also covers the case where an infection with the pest occurs, but is less severe, e.g in terms of the manifestation or duration of symptoms. Preferably, in the context of the present invention a plant is considered to be resistant to a pest if the susceptibility to the pest is reduced by at least 10%, 20%, 30%, 40% or 50%, further preferred reduced by at least 60%, 70%, 80% or 85%, especially preferred reduced by at least 90%, 95%, 98% or 99%, compared to a susceptible wildtype plant or plant population.

The term "host-induced gene silencing" (HIGS) refers to the silencing of a pathogen gene via RNA interference (RNAi, see e.g. Fire et al. 1998, Potent and specific genetic interference by double-stranded RNA in Caenorhabditis elegans, Nature 391, 806-811, doi:10.1038/35888; Koch A and Kogel KH 2014, New wind in the sails: improving the agronomic value of crop plants through RNAi-mediated gene silencing, Plant Biotechnology Journal, doi: 10.1111/pbi.12226; Younis A et al., 2014, RNA Interference (RNAi) Induced Gene Silencing: A Promising Approach of Hi-Tech Plant Breeding. International Journal of Biological Sciences, 10(10), 1150-1158. doi:10.7150/ijbs.10452; WO 2006/070227 A2). RNAi involves e.g. the expression of a double-stranded RNA (dsRNA), small interfering RNA (siRNAs) or micro RNA (miRNA) in the host plant, which subsequently enter the pathogen and trigger posttranscriptional gene silencing (PTGS), e.g. by degradation of target mRNA, or transcriptional gene silencing (TGS), e.g. by epigenetic modifications of the nuclear genome.

The term "gene silencing", as used herein, means reducing the expression level of a target gene compared to the expression level of the wildtype gene. The term encompasses the downregulation or knockdown of a gene on the level of transcription or translation. In particular, gene silencing refers to a reduction of gene expression by at least 50%, 55% or 60%, preferably by at least 70%, 75%, 80%, 85% or at least 90%.

As used herein, the term "dsRNA" refers to two complementary RNA molecules that have annealed to one-another to form a double-stranded RNA molecule. The two strands may comprise the "sense" and "antisense" RNAs of a gene, and may be separated by a coding or non-coding spacer sequence, such that a hairpin structure is formed.

The term "genetically engineering a plant in a manner to enable host-induced gene silencing" relates to biotechnological measures manipulating the plant genetically in such a way that the plant is able to mediate the silencing of a gene of a pathogen, e.g. by producing dsRNA, when in contact with the pathogen. The term "genetically engineering" is not restricted to a manipulation of nuclear DNA, but encompasses manipulation of e.g. extranuclear DNA or the introduction of non-chromosomal genetic elements, e.g. plasmids.

The terms "having the amino acid sequence" or "having the nucleotide sequence" refers to a protein or nucleic acid consisting only of the amino acids or the nucleotides in the given sequence, i.e. without any further amino acids or nucleotides before or after the sequence.

The term "comprising" as used herein encompasses the term "having", i.e. is not to be construed as meaning that further elements have necessarily to be present in an embodiment in addition to the element explicitly mentioned. For example, the term "protein comprising an amino acid sequence according to SEQ ID NO:X" also encompasses a protein having the amino acid sequence according to SEQ ID NO:X, "having" in this context meaning being exclusively composed of the amino acids in SEQ ID NO:X.

The term "x % sequence identity" with regard to a protein or a nucleic acid means a percentage of identical amino acids or nucleotides in two compared sequences when properly aligned. Identity means that when comparing two sequences at equivalent positions the same nucleotide or amino acid is present. It may optionally be necessary to take sequence gaps into account in order to produce the best possible alignment. To determine the degree of identity between two nucleic acids it is preferable to take a minimum length of 60 nucleotides or base pairs, preferably a minimum length of 70, 80, 90, 100, 110, 120, 140, 160, 180, 200, 250 , 300, 350 or 400 nucleotides or base pairs, or a length of at least 25%, 30%, 40%, 50%, 60%, 70%, 80%, 90%, 91%, 92%, 93%, 94%, 95%, 96%, 97%, 98%, 99%, 99.2% or 99.5% of the nucleotides in the respective nucleotide sequences. For proteins it is preferable to take a minimum length of 20, preferably a minimum length of 25, 30, 35, 40, 45, 50, 60, 80 or 100, more preferably a minimum length of 120, 140, 160, 180 or 200 amino acids, or a minimum length of 25%, 30%, 40%, 50%, 60%, 70%, 80%, 90%, 91%, 92%, 93%, 94%, 95%, 96%, 97%, 98%, 99%, 99.2% or 99.5% of the amino acids of the respective amino acid sequences compared. Particularly preferably the full length of the respective protein(s) or nucleic acid(s) is used for comparison. The degree of similarity or identity of two sequences can, for example, be determined by using the computer program BLAST (Altschul, SF et al, 1990, Basic local alignment search tool, J Mol Biol 215:403-410, see, e.g. http://www.ncbi.nlm.nih.gov/BLAST/) using standard parameters. The skilled person, using his expert knowledge, will readily determine which of the available BLAST programs, eg BLASTp or PLASTn, is suitable.

The term "hybridization" is used herein in reference to the pairing of complementary nucleic acids. Hybridization and the strength of hybridization (i.e., the strength of the association between the nucleic acids) is influenced by such factors as the degree of complementarity between the nucleic acids, stringency of the conditions involved, the Tm ("melting temperature") of a nucleic acid of the formed hybrid, and the G:C ratio within the nucleic acids.

The term "hybridizing under stringent conditions" refers to conditions of high stringency, i.e. in term of temperature, ionic strength, and the presence of other compounds such as organic solvents, under which nucleic acid hybridizations are conducted. With "high stringency" conditions, nucleic acid base pairing will occur only between nucleic acids having a high frequency of complementary base sequences. Stringent hybridization conditions are known to the skilled person (see e.g. Green M.R., Sambrook, J., Molecular Cloning: A Laboratory Manual, Cold Spring Harbor Laboratory Press; 4th edition, 2012). An example for stringent hybridization conditions is hybridizing at 42° C in a solution consisting of 5× SSPE (43.8 g/l NaCl, 6.9 g/l NaH₂PO₄H₂O and 1.85 g/l EDTA, pH adjusted to 7.4 with NaOH), 0.5% SDS, 5 × Denhardt's reagent and 100 µg/ml denatured salmon sperm DNA followed by washing in a solution comprising 0.1 × SSPE, 1.0% SDS at 42° C when a probe of about 500 nucleotides in length is employed.

The term "introducing into the plant" in respect of a DNA comprises the introduction of the DNA into the plant resulting in a stable integration of the DNA into the plant genome, such that the DNA can be expressed in the plant, or the introduction of the DNA into the plant in a manner that does not result in stable integration of the DNA into the plant genome, but in a transient expression of the DNA. Suitable methods and means, e.g. plasmid vectors, for introducing DNA into plants are known to the skilled person. Transient expression vectors are known to the skilled person and, e.g., described in Hellens RP et al., 2005, Transient expression vectors for functional genomics, quantification of promoter activity and RNA silencing in plants, Plant Methods 1:13, doi:10.1186/1746-4811-1-13.

The term "reverse complement" refers to the inversely complementary sequence of a nucleotide sequence, i.e. to a sequence complementary to a given sequence in reverse order of the nucleotides. As an example, the reverse complement of a nucleotide sequence having the sequence atggttc is gaaccat.

The term "antisense" refers to RNA sequences that are complementary to a specific mRNA sequence. The term "antisense strand" refers to a nucleic acid strand that is complementary to the "sense" strand. Antisense RNA ("asRNA") molecules are single-stranded nucleic acids which can combine with a sense strand or mRNA to form duplexes due to complementarity of the sequences.

The term "a nucleotide sequence encoding protein x", or the like, refers to any nucleotide sequence coding for protein x with a given amino acid sequence according to the genetic code, and under consideration of the degeneracy of the genetic code.

The term "ortholog" refers to either of two or more homologous genes or proteins found in different species related to each other by linear descent.

The term "homologous" as used herein in reference to a nucleic acid, protein or peptide means that a nucleic acid is in its nucleotide sequence essentially identical or similar to another nucleic acid, or a protein or peptide is in its amino acid sequence essentially identical or similar to another protein or peptide, without being completely identical to the nucleic acid or protein or peptide with which it is compared. The presence of homology between two nucleic acids or proteins or peptides can be determined by comparing a position in the first sequence with a corresponding position in the second sequence in order to determine whether identical or similar residues are present at that position. Two compared sequences are homologous to each other when a certain minimum percentage of identical or similar nucleotides or amino acids are present. Identity means that when comparing two sequences at equivalent positions the same nucleotide or amino acid is present. It may optionally be necessary to take sequence gaps into account in order to produce the best possible alignment. Similar amino acids are non-identical amino acids with the same or equivalent chemical and/or physical properties. The replacement of an amino acid with another amino acid with the same or equivalent physical and/or chemical properties is called a "conservative substitution". Examples of physicochemical properties of an amino acid are hydrophobicity or charge. In connection with nucleic acids it is referred to a similar nucleotide or a conservative substitution when, in a coding sequence, a nucleotide within a codon is replaced with another nucleotide, the new codon, e.g. due to the degeneracy of the genetic code, still encoding the same or a similar amino acid. The skilled person knows which nucleotide or amino acid substitution is a conservative substitution. To determine the degree of similarity or identity between two nucleic acids it is preferable to take a minimum length of 60 nucleotides or base pairs, preferably a minimum length of 70, 80, 90, 100, 110, 120, 140, 160, 180, 200, 250 , 300, 350 or 400 nucleotides or base pairs, or a length of at least 25%, 30%, 40%, 50%, 60%, 70%, 80%, 90%, 91%, 92%, 93%, 94%, 95%, 96%, 97%, 98%, 99%, 99.2% or 99.5% of the nucleotides in the respective nucleotide sequences. For proteins/peptides it is preferable to take a minimum length of 20, preferably a minimum length of 25, 30, 35, 40, 45, 50, 60, 80 or 100, more preferably a minimum length of 120, 140, 160, 180 or 200 amino acids, or a minimum length of 25%, 30%, 40%, 50%, 60%, 70%, 80%, 90%, 91%, 92%, 93%, 94%, 95%, 96%, 97%, 98%, 99%, 99.2% or 99.5% of the amino acids of the respective amino acid sequences compared. Particularly preferably the full length of the respective protein(s) or nucleic acid(s) is used for comparison. The degree of similarity or identity of two sequences can, for example, be determined by using the computer program BLAST (Altschul SF et al., 1990, Basic local alignment search tool, J Mol Biol 215:403-410), see, e.g. http://www.ncbi.nlm.nih.gov/BLAST/) using standard parameters. A determination of homology is dependent on the length of the sequences being compared. For the purposes of the present invention two nucleic acids, the shorter of which comprises at least 100 nucleotides, will be considered homologous when at least 70%, at least 75%, at least 80%, at least 85%, at least 90%, at least 95%, at least 96% , at least 97%, at least 98%, at least 99%, at least 99.2% or 99.5% of the nucleotides are identical and/or similar ("identities" or "positives" according to BLAST), preferably identical. In case of a sequence length of 50-99 nucleotides two nucleic acids are considered homologous when at least 80%, preferably at least 85%, 86%, 87%, 88%, 89%, or 90% of the nucleotides are identical and/or similar. In case of a sequence length of 15-49 nucleotides two nucleic acids are considered homologous when at least 90%, preferably at least 95%, 96%, 97%, 98%, 99%, 99.2% or 99.5% of the nucleotides are identical and/or similar. In the case of nucleic acids coding for a protein or peptide homology is assumed to exist if the translated amino acid sequences are homologous. As similar amino acids especially those non-identical amino acids are considered, which, on the basis of the computer program "Basic Local Alignment Search Tool", abbreviated as BLAST (Altschul SF et al., 1990, Basic local alignment search tool, J Mol Biol 215:403-410); see e.g. http://www.ncbi.nlm.nih.gov/BLAST/) using the BLOSUM62 substitution matrix (Henikoff, S. and Henikoff, J. Amino acid substitution matrices from protein blocks. Proc Natl. Acad. Sci. USA 89: 10915-10919, 1992) are designated as "positive", i.e. have a positive score in the BLOSUM62 substitution matrix. For the purposes of the present invention, it is assumed that a homology between two amino acid sequences is present if at least 55%, preferably at least 60%, at least 65%, at least 70%, at least 75%, at least 80%, at least 85%, at least 90%, at least 95%, at least 97%, at least 98%, at least 99%, at least 99.2% or at least 99.5% of the amino acids are identical or similar, preferably identical. In particular, a homology between two sequences is assumed to exist, when, using the computer program BLAST (Altschul SF et al., 1990, Basic local alignment search tool, J Mol Biol 215:403-410); see, e.g. http://www.ncbi.nlm.nih.gov/BLAST/) using standard parameters and the BLOSUM62 substitution matrix (Henikoff S and Henikoff J, 1992, Amino acid substitution matrices from protein blocks, Proc Natl Acad Sci USA 89: 10915-10919) an identity or similarity ("positives"), preferably identity, of at least 55%, preferably at least 60%, at least 65%, at least 70%, at least 75%, at least 80%, at least 85%, at least 90%, at least 95%, at least 97%, at least 98%, at least 99%, at least 99.2% or at least 99.5% is obtained. The skilled person, using his expert knowledge, will readily determine which of the available BLAST programs, eg BLASTp or BLASTn, is suitable for determination of homology. In addition, the skilled person is aware of further programs for assessing homology, which he may use if necessary. Such programs are, for example, available on the website of the European Bioinformatics Institute (EMBL) (see, e.g http://www.ebi.ac.uk/Tools/similarity.html). Where such terms like "x % homologous to" or "homology of x %" are used herein, this is to be construed as meaning that two proteins or nucleic acids are considered homologous and have a sequence similarity or identity, preferably identity, of x %, e.g. 80%.

The term "fragment" or "part" in relation to a protein refers to a portion of a protein consisting of contiguous amino acids of the protein, preferably at least 10, 15, 20, 25, 30, 35, 40, 45, 50, 55 or at least 60, 70, 80, 90 or 100 contiguous amino acids. In reference to a nucleic acid the term "fragment" or "part" means a portion of the nucleic acid consisting of contiguous nucleotides of the nucleic acid, preferably at least 15, 20, 25, 30, 35, 40, 45, 50, 55 or at least 60, 70, 80, 90, 100, 110, 120, 130, 140 or 150 contiguous nucleotides.

According to the invention, a plant can be genetically engineered by any method suitable to enable host-induced gene silencing of a *Fusarium* gene encoding CSX1. Such methods are known to the skilled person. The amino acid sequence of CSX1 is given in SEQ ID NO:2, the genomic coding sequence (exons only) is given in SEQ ID NO:1.

In a preferred embodiment of the method of the invention the plant is genetically engineered in such a manner that the plant expresses a double-stranded RNA (dsRNA) comprising at least part of the coding sequence of CSX1, the base thymine being, of course, replaced with uracil. For this purpose, a double-stranded first DNA and a double-stranded second DNA may be introduced into the plant, wherein the nucleotide sequences of the coding strands of the first and second DNA are completely or partially reverse complements of each other, such that a double-stranded RNA can be produced therefrom, and wherein the first DNA comprises (i) a nucleotide sequence according to SEQ ID NO: 1, or (ii) a fragment of at least 15, 16, 17, 18, 19, 20, 21, 22, 23, 24, 25, 30, 35, 40, 45 or 50 contiguous nucleotides of a nucleotide sequence according to SEQ ID NO: 1, or (iii) a nucleotide sequence encoding CSX1 according to SEQ ID NO: 2, or (iv) a nucleotide sequence encoding a protein sharing at least 55%, 60%, 65%, 70%, 75%, 80%, 85%, 90%, 95%, 96%, 97%, 98% or 99% sequence identity with the amino acid sequence of SEQ ID NO: 2, or (v) a nucleotide sequence complementary to one of the nucleotide sequences of (i) to (iv), or (vi) a nucleotide sequence hybridizing under stringent conditions with a nucleotide sequence according to one of the nucleotide sequences of (i) to (v).

The first and second double-stranded DNA may be stably integrated into the nuclear genome of the plant, i.e. into a plant chromosome, or integrated into an extra-nuclear element. The first and second DNA may also be integrated into separate extra-nuclear elements. Both the first and second double-stranded DNA comprise a coding and non-coding strand, wherein the coding strand of the second DNA is at least partially reverse-complementary to the coding strand of the first DNA. Transcription of both the first and second DNA leads to the formation of a duplex RNA. Transcription of the first and second DNA may lead to a single RNA strand forming a duplex by intramolecular hybridization, or to two separate RNA strands, wherein the RNA duplex is formed by intermolecular hybridization of the separate RNA strands.

Alternatively, or additionally, the plant may be genetically engineered in a manner to cause the plant to produce an RNA in antisense orientation to a CSX1 mRNA. For this purpose, a double-stranded DNA comprising in its coding strand at least part of the complementary strand of the CSX1 gene may, for example, be stably integrated into the plant genome, be it into the nuclear genome or an extra-nuclear element. Transcription of the DNA leads to the production of antisense mRNA hybridizing at least partially with CSX1 mRNA, resulting in a silencing of the CSX1 gene.

A further alternative or additional measure may consist in genetically engineering the plant in order to cause over-expression of a plant CSX1 ortholog. It is known that over-expression of a gene may lead to the silencing of that gene via RNAi (see e.g. Napoli C et al., 1990, Introduction of a chimeric chalcone synthase gene in petunia results in reversible co-suppression of homologous genes in trans, Plant Cell 2, 279-289). Over-expression of a plant CSX1 ortholog can e.g. be accomplished by introducing into the plant genome one or more further copies of the gene, e.g. under the control of the native or a different, stronger and/or constitutive promoter.

In a preferred embodiment the method of the invention confers resistance against a plant disease caused by a *Fusarium graminearum*. Preferably, the plant disease is Fusarium head blight (FHB).

The plant to which resistance is conferred is preferably a cereal, e.g. wheat (Triticum aestivum), durum wheat (Triticum durum), barley (Hordeum vulgare), oat (Avena sativa), rye (Secale cereale), rice (Oryza sativa) or maize (Zea mays).

In a second aspect the invention relates to a transgenic plant, or parts thereof, with resistance against a plant disease caused by a *Fusarium* species, the plant being genetically engineered in a manner to enable host-induced gene silencing of a *Fusarium* gene encoding CSX1 having the amino acid sequence of SEQ ID NO: 2, or host-induced gene silencing of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

In a preferred embodiment the invention relates to a transgenic plant, or part thereof,
a. in the genome of which a double-stranded first DNA and a double-stranded second DNA has been transiently or stably integrated, wherein the nucleotide sequences of the coding strands of the first and second DNA are completely or partially reverse complements of each other, such that a double-stranded RNA can be produced therefrom, and wherein the first DNA comprises (i) a nucleotide sequence according to SEQ ID NO: 1, or (ii) a fragment of at least 15, 16, 17, 18, 19, 20, 21, 22, 23, 24, 25, 30, 35, 40, 45 or 50 contiguous nucleotides of a nucleotide sequence according to SEQ ID NO: 1, or (iii) a nucleotide sequence encoding CSX1 according to SEQ ID NO: 2, or (iv) a nucleotide sequence encoding a protein sharing at least 55%, 60%, 65%, 70%, 75%, 80%, 85%, 90%, 95%, 96%, 97%, 98% or 99% sequence identity with the amino acid sequence of SEQ ID NO: 2, or (v) a nucleotide sequence complementary to one of the nucleotide sequences of (i) to (iv), or (vi) a nucleotide sequence hybridizing under stringent conditions with a nucleotide sequence according to one of the nucleotide sequences of (i) to (v); and/or
b. producing in its cells an RNA in antisense orientation to a CSX1 mRNA; and/or
c. over-expressing in its cells a plant CSX1 ortholog.

Especially preferred, the transgenic plant is a cereal plant, preferably wheat (Triticum aestivum), durum wheat (Triticum durum), barley (Hordeum vulgare), oat (Avena sativa), rye (Secale cereale), rice (Oryza sativa) or maize (Zea mays).

Further, the transgenic plant of the invention preferably has resistance against a plant disease caused by *Fusarium graminearum*, preferably against Fusarium head blight.

In a third aspect the invention relates to a double-stranded RNA (dsRNA), comprising a first RNA strand and a second RNA strand, wherein the first strand comprises (i) a nucleotide sequence corresponding to SEQ ID NO: 1, or (ii) a fragment of at least 15, 16, 17, 18, 19, 20, 21, 22, 23, 24, 25, 30, 35, 40, 45, 50, 55, 60, 65, 70, 80, 90 or 100 contiguous nucleotides of a nucleotide sequence corresponding to SEQ ID NO: 1, with the proviso that T in SEQ ID NO: 1 is replaced with U, and wherein the second strand is complementary to the first strand. The term "a nucleotide sequence corresponding to SEQ ID NO: 1, with the proviso that T in SEQ ID NO: 1 is replaced with U" refers to a nucleotide sequence differing from the sequence of SEQ ID NO: 1 in that thymine (T) is replaced with uracil (U), because in RNA uracil takes the place of thymine. It is self-evident that "corresponding to" also includes that the RNA nucleotides have ribose instead of deoxyribose used in DNA.

A dsRNA according to the invention may e.g. be used in an anti-fungal composition, for triggering environmental RNA interference (eRNAi). Environmental RNAi is a sequence-specific regulation of endogenous gene expression in a receptive organism, e.g. Fusarium, by exogenous double-stranded RNA (dsRNA). In a fourth aspect, the invention therefore relates to a composition comprising a double-stranded RNA according to the third aspect of the invention.

In a fifth aspect the invention relates to the use of a nucleic acid comprising
(i) a nucleotide sequence according to SEQ ID NO: 1, or
(ii) a fragment of at least 15, 16, 17, 18, 19, 20, 21, 22, 23, 24, 25, 30, 35, 40, 45 or 50 contiguous nucleotides of a nucleotide sequence according to SEQ ID NO: 1, or
(iii) a nucleotide sequence encoding CSX1 according to SEQ ID NO: 2, or
(iv) a nucleotide sequence encoding a protein sharing at least 55%, 60%, 65%, 70%, 75%, 80%, 85%, 90%, 95%, 96%, 97%, 98% or 99% sequence identity with the amino acid sequence of SEQ ID NO: 2, or
(v) a nucleotide sequence complementary to one of the nucleotide sequences of (i) to (iv), or
(vi) a nucleotide sequence hybridizing under stringent conditions with a nucleotide sequence according to one of the nucleotide sequences of (i) to (v),
for conferring to a plant resistance against a plant disease caused by a Fusarium species by host-induced gene silencing of a Fusarium gene encoding CSX1 having or comprising the amino acid sequence of SEQ ID NO: 2 or by host-induced gene silencing of a Fusarium gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

Preferably, the plant disease is Fusarium head blight. Further, the plant to which resistance is conferred is preferably a cereal, preferably wheat *(Triticum aestivum),* durum wheat (*Triticum durum*), barley (*Hordeum vulgare*), oat (*Avena sativa*), rye (*Secale cereale*), rice (*Oryza sativa*) or maize (*Zea mays*).

Still further, the invention, in a sixth aspect, relates to the use of a double-stranded RNA according to the third aspect of the invention or the use of a composition according to the fourth aspect of the invention for conferring to a plant resistance against a plant disease caused by a Fusarium species by gene silencing of a *Fusarium* gene encoding CSX1 having or comprising the amino acid sequence of SEQ ID NO: 2 or by gene silencing of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

Preferably, the plant disease is Fusarium Head Blight. Further, the plant to which resistance is conferred is preferably a cereal, preferably wheat (Triticum aestivum), durum wheat (*Triticum durum*), barley (*Hordeum vulgare*), oat *(Avena sativa),* rye *(Secale cereale),* rice (*Oryza sativa)* or maize (*Zea mays*).

In the following the invention is described for illustration purposes only by way of examples and attached figures.

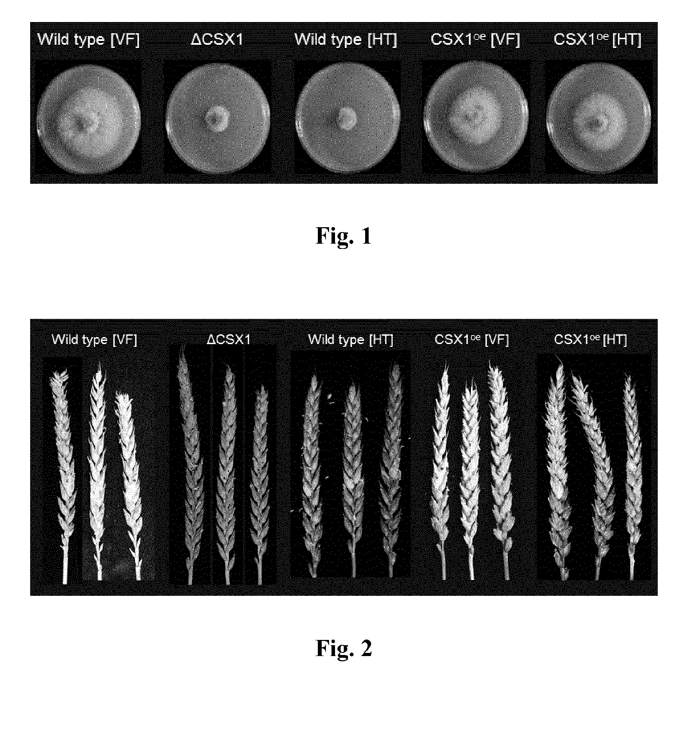

Figure 1. Agar plate assay. Colony morphology of the wild type virus-free (Wild type [VF]) and with high titer of virus (Wild type [HT]), a CSX1 deletion mutant (ΔCSX1, and the mutants expressing CSX1 constitutively, virus free (CSX1^{oe} [VF]) and containing a high titer of virus (CSX1^{oe} [HT]). Wild type [VF] and the CSX1^{oe} mutants grow normal while virus infection of the wild type and CSX1 deletion reduce growth of the fungus.
Figure 2. Pathogencity assay on wheat (cv. Nandu). Wheat heads at mid-anthesis were point inoculated with conidia of the wild type virus-free (Wild type [VF]) and with high titer of virus (Wild type [HT]), a CSX1 deletion mutant (ΔCSX1), and the mutants expressing CSX1 constitutively, virus free (CSX1^{oe} [VF]) and containing a high titer of virus (CSX1^{oe} [HT]). Wild type [VF] and the CSX1^{oe} mutants caused typical Fusarium Head Blight symptoms in the entire spike 21 days post inoculation (dpi) while the wild type [HT] and ΔCSX1 mutants were reduced.

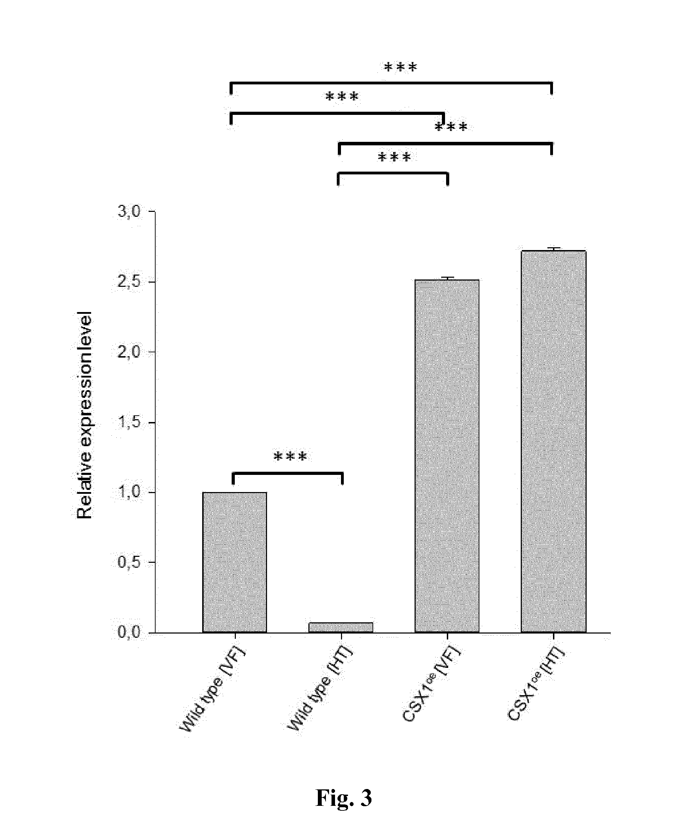

Figure 3. Gene expression analysis of CSX1. Quantitative real-time PCR (qRT-PCR) of CSX1. The relative expression was determined in the wild type virus-free (Wild type [VF]) and with high titer of virus (Wild type [HT]), and the mutants expressing CSX1 constitutively, virus free (CSX1^{oe} [VF] and containing a high titer of virus (CSX1^{oe} [HT]). Transcript levels were normalized to beta-tubulin expression. QRT-PCR was performed using cDNA obtained from samples raised in liquid minimal medium (MM). Error bars indicate the standard deviation. QRT-PCR was performed in triplicates. Asterisks indicate significance according to Pair Wise Fixed Reallocation Randomisation Test (Relative Expression Software Tool; Pfaffl, M. W., Horgan, G. W. and Dempfle, L. (2002) Relative expression software tool (REST©) for group-wise comparison and statistical analysis of relative expression results in real-time PCR. Nucl Acids Res, 30 (9) e36, doi:10.1093/nar/30.9.e36) (p=0.001).

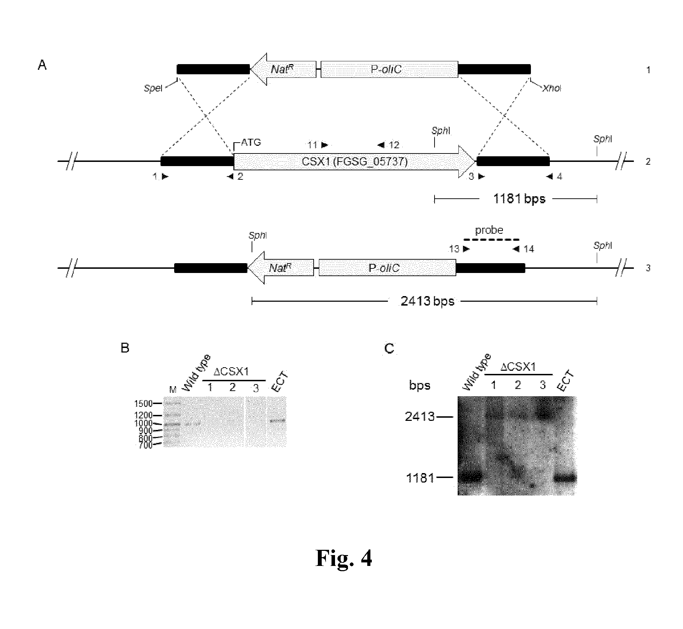

Figure 4. Gene replacement, Southern hybridization and diagnostic PCRs for CSX1. A. Replacement and Southern hybridization strategy for CSX1. Deletion of CSX1 (2) by homologous recombination using a replacement fragment excised from pRS426:deltaCSX1 using restriction enzyme SpeI and XhoI (1) (3: genotype of disrupted strains). Flanking regions are indicated as bold black lines. The gene flanks were fused to a nourseothricin resistance cassette, consisting of the resistance gene (encoding an acetyltransferase), the oliC promoter (P-oliC) from A. nidulans. Primer binding sites for PCR are indicated as small arrows (numbering refers to Table 1). The regions used as probes for Southern analysis is represented by the dashed line. Scheme not to scale. B. PCR analysis of three independent ΔCSX1 and one ectopic mutant, and the wild type. Deletion of CSX1 was verified in the ΔCSX1 mutants (analyzed after single spore purification) using primers #11 (SEQ ID NO: 13) and #12 (SEQ ID NO: 14). The wild type and the ectopic strain (ECT) were PCR-positive for the gene internal fragment (887 bps). C. Southern analysis of ΔCSX1 and the wild type. DNA of the mutants and wild type strain was digested using SphI, separated on agarose gels, blotted on membranes and probed with a DIG-labelled probe for a fragment of the flanking region of CSX1. The probe hybridized with the DNA of the disruption mutants (2413 bps), and the wild type (1181 bps).

### 1. Materials and methods

### 1.1 Fungal strains and culture conditions

All mutants described in this study originate from the *Fusarium graminearum* wild-type strain PH1 (ATCC MYA-4620, NRRL 31084). For vector cloning, the uracil-auxotrophic *S*. *cerevisiae* strain FGSC 9721 (FY 834) was used. Plasmids were amplified using *Escherichia coli* strain DH5α. Bacteria were cultivated in sterile lysogeny broth (LB) medium (Green M.R., Sambrook, J., Molecular Cloning: A Laboratory Manual, Cold Spring Harbor Laboratory Press; 4th edition, 2012) either as a liquid culture or on agar plates, then supplemented with 100 µg ml-1 ampicillin. Yeast cells were cultured in YPG and in SD medium lacking uracil.

For growth assays, mycelial plugs of the wild type and all mutant strains were taken from the edge of a 3-day-old colony on complete medium (CM; Leach, J., Lang, B. R. and Yoder, O. C. (1982) Methods for Selection of Mutants and In Vitro Culture of Cochliobolus heterostrophus. Journal of General Microbiology, 128, 1719-1729.) or minimal medium (MM; as CM but without yeast extract) and placed in the middle of the assay plates. Supplements for the growth assays are indicated in the figure legends. All plates were incubated at 28°C for at least 3 days in the dark. The diameter of the colonies was measured using a technical ruler. The analyses were performed with at least five replicates and using at least two independent mutants.

### 1.2 Vector construction

Standard recombinant DNA methods were performed according to Ausubel et al. (2002) (Ausubel, F. M., Brent, R., Kingston, R. E., Moore, D. D., Seidman, J. G., Smith, J. A., et al. (2002) Short protocols in molecular biology: a compendium of methods from current protocols in molecular biology. New York: Wiley) and Sambrook et al. (1989) (Green M.R., Sambrook, J., Molecular Cloning: A Laboratory Manual, Cold Spring Harbor Laboratory Press; 4th edition, 2012). *Fusarium graminearum* CSX1 was deleted using a double homologous recombination approach. For vector construction, flanking regions (1075 bp upstream and 1047 bp downstream of CSX1) were cloned by PCR from genomic DNA (gDNA) using the primers #1 (SEQ ID NO: 3), #2 (SEQ ID NO: 4), #3 (SEQ ID NO: 5), and #4 (SEQ ID NO: 6) listed in Table 1. The PCR was initiated by denaturation at 94°C for 4 min, followed by 35 cycles of 94°C for 45 s, 55°C for 45 s and 68°C for 60 s. The PCR included a final extension step at 68°C for 10 min and a cooling step at 4°C. The replacement vector was constructed using yeast recombinational cloning method (YRC; Colot, H. V., Park, G., Turner, G. E., Ringelberg, C., Crew, C. M., Litvinkova, L., et al. (2006) A high-throughput gene knockout procedure for Neurospora reveals functions for multiple transcription factors. Proc Natl Acad Sci USA, 103, 10352-10357). For the replacement construct, the gene flanks and a cassette comprising the gene encoding a nourseothricin resistance cassette together with the linearized pRS426 plasmid comprising a ampicillin resistance marker and a gene facilitating uracil biosynthesis were co-transformed into the uracil-auxotrophic yeast strain FGSC 9721 (FY 834). Homologues overhangs to adjacent fragments facilitate recombination and fusion of all fragments to give rise to a circular plasmid that confers uracil prototrophy. Prototrophic clones were selected and checked by PCR (data not shown). Plasmids were isolated from PCR-positive clones and transformed into E. coli. The replacement fragment was released from the plasmid (named pRS426:deltaCSX1) by restriction with *Spe*I and *Xho*I and used for fungal transformation. For generation of overexpression mutants, the entire ORF of CSX1 (SEQ ID NO: 21) was amplified by PCR, ligated into pGEM-T vector (Promega Corp., Madison, WI) and restricted using *Bam*HI restriction sites introduced to the primers #5 (SEQ ID NO: 7) and #6 (SEQ ID NO: 8) (Table 1). The *Bam*HI-released fragment was then ligated into the BamHI-linearized vector p7_GluA containing the constitutive promoter of the glycerinaldehyd-3-phosphate dehydrogenase from *Aspergillus nidulans,* thereby generating plasmid p7GluA:CSX1oe. This vector was inserted into the genome of *F. graminearum* by single crossover after linearization of p7GluA:CSX1oe using *Eco*RV.

### 1.3 Transformation of F. graminearum

A solution of 30-50 µl containing approximately 10 µg DNA was used for protoplast transformation of *F. graminearum.* The protoplast transformation method was performed as described previously (Jansen, C., von Wettstein, D., Schäfer, W., Kogel, K. H., Felk, A. and Maier, F. J. (2005) Infection patterns in barley and wheat spikes inoculated with wild-type and trichodiene synthase gene disrupted Fusarium graminearum. Proc Natl Acad Sci USA, 102, 16892-16897). 100 ml ofYEPD medium (0.3% yeast extract, 1% bacto peptone, 2% D-glucose) was inoculated with 1 × 10⁶ conidia and incubated overnight at 28°C, 150 rpm. The mycelia were collected by filtering with a 200 µm-diameter sieve and washed with double-distilled water. Mycelia were resuspended in a 20 ml mixture of driselase and lysing enzymes (Life Technologies, Darmstadt, Germany; 2.5%: 0.5% in 0.6 M KCl) and incubated for 2-3 h at 30°C, 80 rpm. Undigested hyphal material was removed from the protoplast suspension by filtration. The protoplasts were pelleted by centrifugation at 670 x g, washed once with 10 ml STC (20% sucrose, 10 mM Tris-HCl, pH 8.0, 50 mM CaCl₂), centrifuged again, then resuspended and adjusted in STC at 1x10⁸ protoplasts per ml. For transformation, 200 µl of the protoplast suspension was mixed with DNA. The samples were incubated at room temperature for 20 min. Subsequently, 1 ml PEG (40% polyethylene glycol 4000, 60% STC) was added and again incubated at room temperature for 20 min. The protoplast suspension was added to 5 ml TB3 medium (100 g sucrose, 0.3% yeast extract, 0.3% casamino acids) and shaken overnight at room temperature and 100 rpm for cell wall regeneration. The regenerated protoplasts were pelleted by centrifugation at 4.200 x g, and then mixed with TB3 agar (1.5%) at 50°C with hygromycin B (250 µg ml⁻¹). The mixture was then plated out on petridishes (10 ml/plate). After 24 h, an overlay comprising of TB3 agar (1.5%) and 500 µg ml-1 hygromycin B or 200 µg ml-1 nourseothricin was added to the plates. Putative transformants were obtained after 2 dpi at 28°C. They were transferred to fresh plates of CM supplemented with 250 µg/ml hygromycin B or 100 µg ml-1 nourseothricin and incubated at 28°C. All transformants were purified by single-spore isolation and subsequently checked by diagnostic PCR using gene-internal primers #11 (SEQ ID NO: 13) and #12 (SEQ ID NO: 14) (Table 1).

### 1.4 Southern blot analysis

For southern hybridization analysis, approximately 3 µg of genomic DNA of the wild type, the CSX1 deletion strains and the ectopic mutant strains was restricted with *Sph*I overnight (figure 4). The digested DNA was then separated on 0.8% agarose gels by electrophoresis at 70 V for 6-7 h. Then, the DNA was transferred by capillary blotting onto Hybond NX membranes (GE Healthcare, Munich, Germany), then hybridized with DIG (digoxygenin)-labelled (Roche, Penzberg, Germany) DNA probe amplified from the right flank of the deletion construct (primers #13, SEQ ID NO: 15, and #14, SEQ ID NO: 16, Table 1, figure 4). Detection and visualization procedures were carried out following the manufacturer's manual (Roche).

### 1.5 Virulence assays on wheat

The susceptible spring wheat cultivar Nandu (Lochow-Petkus, Bergen-Wohlde, Germany) was used for wheat virulence assays. Plants were cultivated in a growth room at 20°C with a photoperiod of 16 h and 60% relative humidity, then transferred to infection chambers with optimized conditions. A suspension of 500 conidia in 10 µl of the wild type and all mutants was inoculated into each of two central spikelets at the early stages of anthesis (Jenczmionka N.J., Schäfer W. (2005) The Gpmk1 MAP kinase of Fusarium graminearum regulates the induction of specific secreted enzymes. Current Genetics 47: 29-36.. The inoculated spikes were enclosed in small plastic bags misted with water for the first 3 days, and then monitored for up to three weeks in the infection chambers. Wheat spikes inoculated with 10 µl pure water were used as the negative control. Wheat infection assays were repeated 30 times for each strain.

### 1.6 Expression analysis by quantitative real-time PCR

For gene expression analysis, RNA was isolated using TriFast (Peqlab, Erlangen, Germany). For in-vitro analyses, mycelia were grown in YPG for three days, then shifted to liquid CM and harvested after 30 min of induction. Prior RNA isolation, the samples were frozen in liquid nitrogen and freeze-dried overnight. For RT-PCR, SuperScript II RNase H- Reverse Transcriptase (Themo Scientific, Schwerte, Germany) was used, according to the manufacturer's instructions. The resulting single-stranded cDNA was later used as a template for quantitative real-time PCR (qRT-PCR) reactions. Transcript levels of the target genes were normalized against beta-tubulin gene expression (primers #15, SEQ ID NO: 17, and #16, SEQ ID NO: 18). The qRT-PCR reactions were carried out using gene-specific primers #17 (SEQ ID NO: 19) and #18 (SEQ ID NO: 20) (Table 1), with LightCycler 480 SYBR Green I Master (Roche, Penzberg, Germany) in a volume of 20 µl in a light Cycler 480 (Roche, Penzberg, Germany). The PCR program was as follows: incubation for 2 min at 50 °C, then 2 min at 95 °C, followed by up to 40 cycles of denaturation at 94 °C for 30 s, annealing at 55-58 °C for 30 s and extension at 72 °C for 15 s, followed by a melting curve analysis in order to check the specificity of fragment amplification. All of the measurements were repeated twice, each with three replicates. Relative changes in gene expression were calculated using the comparative Cp method (using REST software (Pfaffl, M. W., Horgan, G. W. and Dempfle, L. (2002) Relative expression software tool (REST©) for group-wise comparison and statistical analysis of relative expression results in real-time PCR. Nucl Acids Res, 30, e36-e36).

**Table 1. Primer list (in 5'-3' direction, underlined are restriction enzyme recognition sequences introduced into the primers)**

| No. | Sequence |
|---|---|
| 1 | GGCCCCCCCTCGAGGTSGACGGTATCGATGGACGCTGGATCTCAAACCTT (SEQ ID NO: 3) |
| 2 | CCGGCTCCAGCGCCTGCACCAGCTCCATACTCCTAATTTGTCAGTACGTC (SEQ ID NO: 4) |
| 3 | GCTTCCAAGCGGAGCAGGCTCGACGTTATTACAAAGCTGCACGCAGTAATA (SEQ ID NO: 5) |
| 4 | GCTCTAGAACTAGTGGATCCCCCGGGCTGATCGCGGAGAAGCAAATTACG (SEQ ID NO: 6) |
| 5 | GGATCCATGAACCCTGGTTTCTCTGGTG (SEQ ID NO: 7) |
| 6 | GGATCCTTAATACTCCTAATTTGTCAGTACG (SEQ ID NO: 8) |
| 7 | GGATCCATGGCATCGAACGCATTGTTCG (SEQ ID NO: 9) |
| 8 | AAGCTTGGCTGCAGGTCGACGGTTAACGGTCTT (SEQ ID NO: 10) |
| 9 | ACTAGTCGGGATAGTTCCGACCTAG (SEQ ID NO: 11) |
| 10 | CTCGAGTTATATAGATGTTCAGCTAT (SEQ ID NO: 12) |
| 11 | GATCTGGCCCCGACTCTCC (SEQ ID NO: 13) |
| 12 | GGTAGCCCTGCATCTGGTT (SEQ ID NO: 14) |
| 13 | TTTATGAGCGATACCCAACCT (SEQ ID NO: 15) |
| 14 | AACACAACACTCAATCATAGCC (SEQ ID NO: 16) |
| 15 | TGTCGACGACCAGTTCTCAGC (SEQ ID NO: 17) |
| 16 | CGATGTCGGCGTCTTGGTAT (SEQ ID NO: 18) |
| 17 | GCTATGTCATCTACCGTGTC (SEQ ID NO: 19) |
| 18 | GGTTGATCCAGTAAGAGTTGAG (SEQ ID NO: 20) |

### 2. Results

Fungal infection by the mycovirus FgV-Ch9 negatively affects several functions in the fungal life cycle. Wild type mycelia containing a high titer of virus (wild type [HT]) show a markedly reduced hyphal growth on agar medium when compared to the non-infected wild type (wild type [VF]) (figure 1, see also Darissa, O., Willingmann, P., Schäfer, W. and Adam, G. (2011) A novel double-stranded RNA mycovirus from Fusarium graminearum: nucleic acid sequence and genomic structure. Archives of virology 156, 647-658, and Darissa, O., Adam, G. and Schäfer, W. (2012) A dsRNA mycovirus causes hypovirulence of Fusarium graminearum to wheat and maize. European Journal of Plant Pathology 134, 181-189). Virulence on wheat is drastically reduced in wild type [HT] (figure 2). Spread of infection is restricted to the point-inoculated wheat spikelet (figure 2).

We identified the potentially mRNA-binding protein FGSG_05737 (named CSX1) as a central regulator of the fungal response to virus infection. Quantitative RT-PCR revealed a 15-fold downregulation of CSX1 in wild type [HT] compared to wild type [VF] (figure 3).

For functional characterization of CSX1, we constructed deletion and over expression mutants of CSX1. Gene deletion was accomplished by double homologues recombination using gene-homologues flanks enclosing a resistance cassette. In two separate transformation events, in total three homokaryotic deletion mutants (ΔCSX1) were generated (verified by PCR and Southern blot analysis; figure 4). For overexpression of CSX1, the entire open reading frame was put under control of the glyceraldehyd-3-phosphate dehydrogenase (*gpdA*) promoter of *A*. *nidulans* that facilitates high expression rates in *F. graminearum* (Nguyen, T. V., Kröger, C., Bonnighausen, J., Schäfer, W. and Bormann, J. (2013) The ATF/CREB transcription factor Atfl is essential for full virulence, deoxynivalenol production and stress tolerance in the cereal pathogen Fusarium graminearum. Mol Plant Microbe Interact, 26, 1378-1394). Successful integration and, therefore, over expression of CSX1 was verified by qRT-PCR using gene-specific primers #17 (SEQ ID NO: 19) and #18 (SEQ ID NO: 20) (figure 3).

Targeted gene deletion of CSX1 renders the fungus, to a certain degree to the same extent like FgV-Ch9 infection, impaired in growth and virulence, further substantiating the assumption of a functional link between virus infection, CSX1 expression and fungal fitness. When grown on solid medium (MM and CM), ΔCSX1 displayed a retarded growth (3 independent mutants tested; figure 1). The median colony diameter was approximately 40% of the wild type colonies. Furthermore, ΔCSX1 mutants were similarly retarded in virulence on wheat as it was shown for the wild type [HT]. The ΔCSX1 mutants caused less Fusarium head blight symptoms compared to wild type [VF] (figure 2).

Interestingly, vertical transmission of FgV-Ch9 via anastomoses failed permanently for ΔCSX1. The CSX1^{oe} mutants, in contrast, got readily infected, but, strikingly, this did not cause any symptoms in regard to vegetative growth (figure 1) and virulence (figure 2), although accumulation of viral dsRNA is comparable to the virus-infected wild type. In regard to growth habit and virulence, the CSX1^{oe} [HT] mutants were indistinguishable from wild type [VF] and CSX1^{oe} [VF] which alone seems not to show any obvious phenotype in regard to vegetative growth (figure 1) and virulence (figure 2). The CSX1^{oe} mutants showed a 2.5 (CSX1^{oe} [VF]) and 2.7 (CSX1 [HT]) fold higher expression of CSX1 compared to wild type [VF] and even approximately 36 and 39 fold higher expression when compared to the wild type [HT], respectively (figure 3). Hence, a specific up-regulation of CSX1 alone is sufficient to prevent the pleiotropic phenotypes due to a virus infection and leads to a symptomless FgV-Ch9 infection of *F*. *graminearum*.

Overview of sequences (aa = number of amino acids, bp = number of base pairs, PRT = protein, nt = number of nucleotides):

| SEQ ID NO: | Type | aa | bp/nt | description |
|---|---|---|---|---|
| 1 | DNA | | 1230 | CSX1 coding sequence (i.e. exons only) |
| 2 | PRT | 409 | | CSX1 protein |
| 3 | DNA | | 50 | primer #1 |
| 4 | DNA | | 50 | primer #2 |
| 5 | DNA | | 51 | primer #3 |
| 6 | DNA | | 50 | primer #4 |
| 7 | DNA | | 28 | primer #5 |
| 8 | DNA | | 31 | primer #6 |
| 9 | DNA | | 28 | primer #7 |
| 10 | DNA | | 33 | primer #8 |
| 11 | DNA | | 25 | primer #9 |
| 12 | DNA | | 26 | primer # 10 |
| 13 | DNA | | 19 | primer # 11 |
| 14 | DNA | | 19 | primer # 12 |
| 15 | DNA | | 21 | primer # 13 |
| 16 | DNA | | 22 | primer # 14 |
| 17 | DNA | | 21 | primer # 15 |
| 18 | DNA | | 20 | primer # 16 |
| 19 | DNA | | 20 | primer # 17 |
| 20 | DNA | | 22 | primer # 18 |
| 21 | DNA | | 1371 | CSX1, genomic sequence (with introns) |

## Claims

1. A method of conferring to a plant resistance against a plant disease caused by a *Fusarium* species, the method comprising genetically engineering the plant in a manner to enable host-induced gene silencing of a *Fusarium* gene encoding CSX1 having or comprising the amino acid sequence of SEQ ID NO: 2 or host-induced gene silencing of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

2. The method according to claim 1, comprising:
a. introducing into the plant a double-stranded first DNA and a double-stranded second DNA, wherein the nucleotide sequences of the coding strands of the first and second DNA are completely or partially reverse complements of each other, such that a double-stranded RNA can be produced therefrom, and wherein the first DNA comprises (i) a nucleotide sequence according to SEQ ID NO: 1, or (ii) a fragment of at least 15, 16, 17, 18, 19, 20, 21, 22, 23, 24, 25, 30, 35, 40, 45 or 50 contiguous nucleotides of a nucleotide sequence according to SEQ ID NO: 1, or (iii) a nucleotide sequence encoding CSX1 according to SEQ ID NO: 2, or (iv) a nucleotide sequence encoding a protein sharing at least 55%, 60%, 65%, 70%, 75%, 80%, 85%, 90%, 95%, 96%, 97%, 98% or 99% sequence identity with the amino acid sequence of SEQ ID NO: 2, or (v) a nucleotide sequence complementary to one of the nucleotide sequences of (i) to (iv), or (vi) a nucleotide sequence hybridizing under stringent conditions with a nucleotide sequence according to one of the nucleotide sequences of (i) to (v); and/or
b. causing the plant to produce an RNA in antisense orientation to a CSX1 mRNA; and/or
c. over-expressing a plant CSX1 ortholog.

3. The method according to claim 1 or 2, wherein the *Fusarium* species is *Fusarium graminearum*.

4. The method according to one of the preceeding claims, wherein the plant to which resistance is conferred is a cereal, preferably wheat (*Triticum aestivum*), durum wheat *(Triticum durum*), barley (*Hordeum vulgare*), oat *(Avena sativa),* rye (*Secale cereale*), rice (*Oryza sativa*) or maize (*Zea mays*).

5. A transgenic plant, or parts thereof, with resistance against a plant disease caused by a *Fusarium* species, the plant being genetically engineered in a manner to enable host-induced gene silencing of a *Fusarium* gene encoding CSX1 having the amino acid sequence of SEQ ID NO: 2 or host-induced gene silencing of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

6. The transgenic plant, or parts thereof, according to claim 5,
a. in the genome of which a double-stranded first DNA and a double-stranded second DNA has been transiently or stably integrated, wherein the nucleotide sequences of the coding strands of the first and second DNA are completely or partially reverse complements of each other, such that a double-stranded RNA can be produced therefrom, and wherein the first DNA comprises (i) a nucleotide sequence according to SEQ ID NO: 1, or (ii) a fragment of at least 15, 16, 17, 18, 19, 20, 21, 22, 23, 24, 25, 30, 35, 40, 45 or 50 contiguous nucleotides of a nucleotide sequence according to SEQ ID NO: 1, or (iii) a nucleotide sequence encoding CSX1 according to SEQ ID NO: 2, or (iv) a nucleotide sequence encoding a protein sharing at least 55%, 60%, 65%, 70%, 75%, 80%, 85%, 90%, 95%, 96%, 97%, 98% or 99% sequence identity with the amino acid sequence of SEQ ID NO: 2, or (v) a nucleotide sequence complementary to one of the nucleotide sequences of (i) to (iv), or (vi) a nucleotide sequence hybridizing under stringent conditions with a nucleotide sequence according to one of the nucleotide sequences of (i) to (v); and/or
b. producing in its cells an RNA in antisense orientation to a CSX1 mRNA; and/or
c. over-expressing in its cells a plant CSX1 ortholog.

7. The transgenic plant, or parts thereof, according to one of claims 5 or 6, wherein the plant is a cereal, preferably wheat *(Triticum aestivum),* durum wheat *(Triticum durum*), barley (*Hordeum vulgare*), oat (*Avena sativa*), rye *(Secale cereale*), rice (*Oryza sativa*) or maize (*Zea mays*).

8. The transgenic plant, or parts thereof, according to one of claims 5 to 7, wherein the plant has resistance against a plant disease caused by *Fusarium graminearum*, preferably against Fusarium Head Blight.

9. Double-stranded RNA, comprising a first RNA strand and a second RNA strand, wherein the first strand comprises (i) a nucleotide sequence corresponding to SEQ ID NO: 1, or (ii) a fragment of at least 15, 16, 17, 18, 19, 20, 21, 22, 23, 24, 25, 30, 35, 40, 45, 50, 55, 60, 65, 70, 80, 90 or 100 contiguous nucleotides of a nucleotide sequence corresponding to SEQ ID NO: 1, with the proviso that T in SEQ ID NO: 1 is replaced with U, and wherein the second strand is complementary to the first strand.

10. Composition, comprising a double-stranded RNA according to claim 9.

11. Use of a nucleic acid comprising
(i) a nucleotide sequence according to SEQ ID NO: 1, or
(ii) a fragment of at least 15, 16, 17, 18, 19, 20, 21, 22, 23, 24, 25, 30, 35, 40, 45 or 50 contiguous nucleotides of a nucleotide sequence according to SEQ ID NO: 1, or
(iii) a nucleotide sequence encoding CSX1 according to SEQ ID NO: 2, or
(iv) a nucleotide sequence encoding a protein sharing at least 55%, 60%, 65%, 70%, 75%, 80%, 85%, 90%, 95%, 96%, 97%, 98% or 99% sequence identity with the amino acid sequence of SEQ ID NO: 2, or
(v) a nucleotide sequence complementary to one of the nucleotide sequences of (i) to (iv), or
(vi) a nucleotide sequence hybridizing under stringent conditions with a nucleotide sequence according to one of the nucleotide sequences of (i) to (v),
for conferring to a plant resistance against a plant disease caused by a *Fusarium* species by host-induced gene silencing of a *Fusarium* gene encoding CSX1 having or comprising the amino acid sequence of SEQ ID NO: 2 or by host-induced gene silencing of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

12. Use of a double-stranded RNA according to claim 9 or a composition according to claim 10 for conferring to a plant resistance against a plant disease caused by a *Fusarium* species by gene silencing of a *Fusarium* gene encoding CSX1 having or comprising the amino acid sequence of SEQ ID NO: 2 or by gene silencing of a *Fusarium* gene encoding a protein sharing at least 55%, 60%, 65%, 70%, 75%, 80%, 85%, 90%, 95%, 96%, 97%, 98% or 99% sequence identity with the amino acid sequence of SEQ ID NO: 2.

13. Use according to one of claims 11 or 12, wherein the plant disease is Fusarium head blight.

14. Use according to one of claims 11 to 13, wherein the plant to which resistance is conferred is a cereal, preferably wheat (*Triticum aestivum*), durum wheat (*Triticum durum*), barley (*Hordeum vulgare*), oat (*Avena sativa*), rye (*Secale cereale*), rice (*Oryza sativa*) or maize (*Zea mays*).


SEQUENCE LISTING

<110> UniversitÃ¤t Hamburg

<120> Method of conferring resistance against a Fusarium plant disease

<130> PAT 1521 EP

<160> 21

<170> BiSSAP 1.3

<210> 1
<211> 1230
<212> DNA
<213> Fusarium

<400> 1
atgaaccctg gtttctctgg tgtgcccgcc aacaacggtg ccccttctgg ccctatggcc 60

tactccaacg gcactcctcc cgcccctgac atgggcggcg ttgcccctcc ccctggatct 120

ggccccgact ctcccaagac caccctttgg atgggtgagc tcgaaccttg gatggacgag 180

aatttcatca agggcgtctt cttgtcttcg gctcatgaaa atgtaaacgt taaggtcatc 240

cgcgacaaga actctggcaa cgccggctac tgtttcgtcg agttccagag ccccgaggct 300

gccaccaagg ccctcaacat gaacggttca caagtcccta atagcagtcg ctccttcaag 360

ctcaactggg cctctggtgg tggtcttgtt gaccgacggg atgatcgcgg ccccgagtac 420

agcattttcg ttggcgacct tggtcccgag gtcaacgagt acgtcctggt ttctctcttc 480

caggctcgtt tcccctcttg caagtctgcc aagatcatga ccgatgctat gagcggtcag 540

agccgcggct acggcttcgt tcgcttctcc gacgagaacg accagcagcg cgccctcgtt 600

gagatgcagg gagtctactg cggtaaccga cccatgcgaa tctctactgc tacccccaag 660

aaccgtggca accacggctt cggtggccaa ggacaccagc atggcggtcc tatgatgccc 720

ggcatgcccc agcagcagat gtggaacggt ggtgggatgc agggcttccc ttatggaggt 780

ttcaaccctg ctacccagat gaaccagttc actgacccca acaacaccac cgtctttgtc 840

ggtggtcttt ccggctacgt caccgaggac gagcttcgat cattcttcca gggctttggc 900

gagatcacct atgttaagat accgcctggc aagggctgcg gttttgtcca gtttgtccac 960

cgccacgccg ccgagatggc catcaaccag atgcagggct accctatcgg taactctcgt 1020

gtccgcctca gctggggtcg atctcagaac aactctggcg tcggcactcc ctaccgccct 1080

gccccccctc ctcctcacta catgggcatg cccagccacg gcggacctgg caactacggc 1140

cctcagcact ttggtggccc tgctcccggt ccccagggac ctcctggacc tcccggcccc 1200

ggccccaacg gccctcctca ggagtattaa 1230

<210> 2
<211> 409

<212> PRT
<213> Fusarium

<220>
<223> CSX1

<400> 2
Met Asn Pro Gly Phe Ser Gly Val Pro Ala Asn Asn Gly Ala Pro Ser
1               5                   10                  15
Gly Pro Met Ala Tyr Ser Asn Gly Thr Pro Pro Ala Pro Asp Met Gly
            20                  25                  30
Gly Val Ala Pro Pro Pro Gly Ser Gly Pro Asp Ser Pro Lys Thr Thr
        35                  40                  45
Leu Trp Met Gly Glu Leu Glu Pro Trp Met Asp Glu Asn Phe Ile Lys
    50                  55                  60
Gly Val Phe Leu Ser Ser Ala His Glu Asn Val Asn Val Lys Val Ile
65                  70                  75                  80
Arg Asp Lys Asn Ser Gly Asn Ala Gly Tyr Cys Phe Val Glu Phe Gln
                85                  90                  95
Ser Pro Glu Ala Ala Thr Lys Ala Leu Asn Met Asn Gly Ser Gln Val
            100                 105                 110
Pro Asn Ser Ser Arg Ser Phe Lys Leu Asn Trp Ala Ser Gly Gly Gly
        115                 120                 125
Leu Val Asp Arg Arg Asp Asp Arg Gly Pro Glu Tyr Ser Ile Phe Val
    130                 135                 140
Gly Asp Leu Gly Pro Glu Val Asn Glu Tyr Val Leu Val Ser Leu Phe
145                 150                 155                 160
Gln Ala Arg Phe Pro Ser Cys Lys Ser Ala Lys Ile Met Thr Asp Ala
                165                 170                 175
Met Ser Gly Gln Ser Arg Gly Tyr Gly Phe Val Arg Phe Ser Asp Glu
            180                 185                 190
Asn Asp Gln Gln Arg Ala Leu Val Glu Met Gln Gly Val Tyr Cys Gly
        195                 200                 205
Asn Arg Pro Met Arg Ile Ser Thr Ala Thr Pro Lys Asn Arg Gly Asn
    210                 215                 220
His Gly Phe Gly Gly Gln Gly His Gln His Gly Gly Pro Met Met Pro
225                 230                 235                 240
Gly Met Pro Gln Gln Gln Met Trp Asn Gly Gly Gly Met Gln Gly Phe
                245                 250                 255
Pro Tyr Gly Gly Phe Asn Pro Ala Thr Gln Met Asn Gln Phe Thr Asp
            260                 265                 270
Pro Asn Asn Thr Thr Val Phe Val Gly Gly Leu Ser Gly Tyr Val Thr
        275                 280                 285
Glu Asp Glu Leu Arg Ser Phe Phe Gln Gly Phe Gly Glu Ile Thr Tyr
    290                 295                 300
Val Lys Ile Pro Pro Gly Lys Gly Cys Gly Phe Val Gln Phe Val His
305                 310                 315                 320
Arg His Ala Ala Glu Met Ala Ile Asn Gln Met Gln Gly Tyr Pro Ile
                325                 330                 335
Gly Asn Ser Arg Val Arg Leu Ser Trp Gly Arg Ser Gln Asn Asn Ser
            340                 345                 350
Gly Val Gly Thr Pro Tyr Arg Pro Ala Pro Pro Pro Pro His Tyr Met
        355                 360                 365
Gly Met Pro Ser His Gly Gly Pro Gly Asn Tyr Gly Pro Gln His Phe
    370                 375                 380
Gly Gly Pro Ala Pro Gly Pro Gln Gly Pro Pro Gly Pro Pro Gly Pro
385                 390                 395                 400
Gly Pro Asn Gly Pro Pro Gln Glu Tyr
                405

<210> 3
<211> 50
<212> DNA

<213> Artificial Sequence

<220>
<223> Primer

<400> 3
ggccccccct cgaggtsgac ggtatcgatg gacgctggat ctcaaacctt          50


<210> 4
<211> 50
<212> DNA
<213> Artificial Sequence

<220>
<223> Primer

<400> 4
ccggctccag cgcctgcacc agctccatac tcctaatttg tcagtacgtc          50


<210> 5
<211> 51
<212> DNA
<213> Artificial Sequence

<220>
<223> Primer

<400> 5
gcttccaagc ggagcaggct cgacgttatt acaaagctgc acgcagtaat a        51


<210> 6
<211> 50
<212> DNA
<213> Artificial Sequence

<220>
<223> Primer

<400> 6
gctctagaac tagtggatcc cccgggctga tcgcggagaa gcaaattacg          50


<210> 7
<211> 28
<212> DNA
<213> Artificial Sequence

<220>
<223> Primer

<220>
<221> misc_feature
<222> 1..6
<223> /note="restriction enzyme recognition sequence"

<400> 7
ggatccatga accctggttt ctctggtg                                  28


<210> 8

<211> 31
<212> DNA
<213> Artificial Sequence

<220>
<223> Primer

<220>
<221> misc_feature
<222> 1..6
<223> /note="restriction enzyme recognition sequence"

<400> 8
ggatccttaa tactcctaat ttgtcagtac g                                    31


<210> 9
<211> 28
<212> DNA
<213> Artificial Sequence

<220>
<223> Primer

<220>
<221> misc_feature
<222> 1..6
<223> /note="restriction enzyme recognition sequence"

<400> 9
ggatccatgg catcgaacgc attgttcg                                        28


<210> 10
<211> 33
<212> DNA
<213> Artificial Sequence

<220>
<223> Primer

<220>
<221> misc_feature
<222> 1..6
<223> /note="restriction enzyme recognition sequence"

<400> 10
aagcttggct gcaggtcgac ggttaacggt ctt                                   33


<210> 11
<211> 25
<212> DNA
<213> Artificial Sequence

<220>
<223> Primer

<220>
<221> misc_feature
<222> 1..6
<223> /note="restriction enzyme recognition sequence"

<400> 11
actagtcggg atagttccga cctag                                          25


<210> 12
<211> 26
<212> DNA
<213> Artificial Sequence

<220>
<223> Primer

<220>
<221> misc_feature
<222> 1..6
<223> /note="restriction enzyme recognition sequence"

<400> 12
ctcgagttat atagatgttc agctat                                         26


<210> 13
<211> 19
<212> DNA
<213> Artificial Sequence

<220>
<223> Primer

<400> 13
gatctggccc cgactctcc                                                 19


<210> 14
<211> 19
<212> DNA
<213> Artificial Sequence

<220>
<223> Primer

<400> 14
ggtagccctg catctggtt                                                 19


<210> 15
<211> 21
<212> DNA
<213> Artificial Sequence

<220>
<223> Primer

<400> 15
tttatgagcg atacccaacc t                                              21


<210> 16
<211> 22
<212> DNA
<213> Artificial Sequence

<220>

<223> Primer

<400> 16
aacacaacac tcaatcatag cc                                                22


<210> 17
<211> 21
<212> DNA
<213> Artificial Sequence

<220>
<223> Primer

<400> 17
tgtcgacgac cagttctcag c                                                 21


<210> 18
<211> 20
<212> DNA
<213> Artificial Sequence

<220>
<223> Primer

<400> 18
cgatgtcggc gtcttggtat                                                   20


<210> 19
<211> 20
<212> DNA
<213> Artificial Sequence

<220>
<223> Primer

<400> 19
gctatgtcat ctaccgtgtc                                                   20


<210> 20
<211> 22
<212> DNA
<213> Artificial Sequence

<220>
<223> Primer

<400> 20
ggttgatcca gtaagagttg ag                                                22


<210> 21
<211> 1371
<212> DNA
<213> Fusarium

<400> 21
atgaaccctg gtttctctgg tgtgcccgcc aacaacggtg ccccttctgg ccctatggcc      60

tactccaacg gcactcctcc cgcccctgac atgggcggcg ttgcccctcc ccctggatct     120

ggccccgact ctcccaagac caccctttgg taagtaaaga tccccaccaa cgcctgcaac 180

gatactgatt gccataggat gggtgagctc gaaccttgga tggacgagaa tttcatcaag 240

ggcgtcttct tgtcttcggc tcatgaaaat gtaaacgtta aggtcatccg cgacaagaac 300

tctggcaacg ccggctactg tttcgtcgag ttccagagcc ccgaggctgc caccaaggcc 360

ctcaacatga acggttcaca agtccctaat agcagtcgct ccttcaagct caactgggcc 420

tctggtggtg gtcttgttga ccgacggtat gtcacccacc tacactgtcg atatcgctta 480

ctgacatacc cagggatgat cgcggccccg agtacagcat tttcgttggc gaccttggtc 540

ccgaggtcaa cgagtacgtc ctggtttctc tcttccaggc tcgtttcccc tcttgcaagt 600

ctgccaagat catgaccgat gctatgagcg gtcagagccg cggctacggc ttcgttcgct 660

tctccgacga gaacgaccag cagcgcgccc tcgttgagat gcagggagtc tactgcggta 720

accgacccat gcgaatctct actgctaccc ccaagaaccg tggcaaccac ggcttcggtg 780

gccaaggaca ccagcatggc ggtcctatga tgcccggcat gccccagcag cagatgtgga 840

acggtggtgg gatgcagggc ttcccttatg gaggtttcaa ccctgctacc cagatgaacc 900

agttcactga ccccaacaac accaccgtct ttgtcggtgg tctttccggc tacgtcaccg 960

aggacgagct tcgatcattc ttccagggct ttggcgagat cacctatgtt aagataccgc 1020

ctggcaaggg ctgcggtttt gtccagtttg tccaccgcca cgccgccgag atggccatca 1080

accagatgca gggctaccct atcggtaact ctcgtgtccg cctcagctgg ggtcgatctc 1140

agaacaactc tggcgtcggc actccctacc gccctgcccc ccctcctcct cactacatgg 1200

gcatgcccag ccacggcgga cctggcaact acggccctca gcactttggt ggccctgctc 1260

ccggtcccca gggacctcct ggacctcccg gccccggccc caacggccct cctcaggtaa 1320

gcatcttggc tctcacgaac cgttgacgta ctgacaaatt aggagtatta a 1371